# EUROPÄISCHE PATENTSCHRIFT

(11) **EP 1 098 940 B1**
(45) Veröffentlichungstag und Bekanntmachung des Hinweises auf die Patenterteilung: **04.09.2002**
(21) Anmeldenummer: 98965281.3
(22) Anmeldetag: 17.12.1998
(51) Int. Cl.: C09D 105/02, C08J 7/04, G01N 30/48

(54) **HYDROPHILE POLYMERBESCHICHTUNGEN AUF HYDROPHOBEN ODER HYDROPHOBISIERTEN OBERFLÄCHEN FÜR BIOTECHNOLOGISCHE ANWENDUNGEN**
HYDROPHILIC POLYMER COATINGS ON HYDROPHOBIC OR HYDROPHOBIZED SURFACES FOR BIOTECHNOLOGICAL APPLICATIONS
REVETEMENTS POLYMERES HYDROPHILES SUR DES SURFACES HYDROPHOBES OU HYDROPHOBISEES POUR APPLICATIONS BIOTECHNOLOGIQUES

(30) Priorität: 17.12.1997 DE 19756193
(43) Veröffentlichungstag der Anmeldung: 16.05.2001
(73) Patentinhaber: Schleicher, Franz, Dr., 85053 Ingolstadt (DE)
(72) Erfinder: BRINK, Gunnar, D-80339 München (DE); GASSNER, Manuela, D-81739 München (DE); GÖTZ, Silke, D-81373 München (DE); WISCHERHOFF, Erik, D-81379 München (DE)
(74) Vertreter: VOSSIUS & PARTNER
(86) Internationale Anmeldenummer: EP9808308
(87) Internationale Veröffentlichungsnummer: WO99031189

(56) Entgegenhaltungen:
- C. FOURNIER ET AL.: "Coating polystyrene particles by adsorption of hydrophobically modified dextran" LANGMUIR, Bd. 11, 1995, Seiten 2344-2347, XP002101627 usa
- DATABASE WPI Week 9650 Derwent Publications Ltd., London, GB; AN 96-502647 XP002101628 "Optical resolution agents used for chromatography fillers - comprise porous carriers contg. polymerised cpds. of polysaccharide derivs. on surface" & JP 08 259470 A (NACALAI TESQUE INC) , 8. Oktober 1996

## Beschreibung

Die vorliegende Erfindung betrifft Beschichtungen aus hydrophilen ungeladenen Polymeren mit hydrophoben Gruppen auf hydrophoben oder hydrophobisierten Oberflächen und Verfahren zu deren Herstellung. Genauer betrifft die Erfindung Beschichtungen der genannten Art für biotechnologische Anwendungen, die eine unspezifische Adsorption von organischem, insbesondere biologischem Material an Geräte-, z.B. Sensoroberflächen verhindern.

Sollen Kunststoffteile in Geräten oder Sensoren zur Anwendung kommen, die mit organischem Material in Kontakt kommen, ist zur Vermeidung unspezifischer Adsorption eine Modifizierung der Oberflächen erforderlich, die bisher zeit- und kostenintensiv war. Unter anderem sind hierfür die im folgenden beschriebenen Verfahren bekannt.

Polymeroberflächen können durch Plasmabehandlung hydrophilisiert werden (J. Chem. Phys. 97, 12835 (1993)). Dabei wird die Oberfläche oxidiert, wodurch Hydroxy- und Carboxylgruppen gebildet werden. Diese Oberflächen besitzen keine sehr definierte Funktionalisierung und sind ohne weitere Behandlung nicht geeignet, unspezifische Adsorption zu verhindern.

In Biomaierials 18, 845 (1997) werden Polystyroloberflächen mit Carboxylgruppen funktionalisiert, indem zunächst eine Adsorption eines Salzes der Undecylensäure erfolgt, der sich eine Argonplasmabehandlung anschließt. Hierbei entsteht eine geladene Oberfläche, die zwar hydrophil ist, aber über elektrostatische Wechselwirkungen erneut zu unspezifischer Adsorption neigt.

Eine weitere Behandlungsmöglichkeit besteht in der Adsorption von Polyelektrolyten (Macromolecules 30, 1752-1757 (1997)) auf oxidativ vorbehandelten Oberflächen. Auch hier entstehen geladene Oberflächen mit den oben beschriebenen Nachteilen.

Die Adsorption von Polyelektrolyten kann ohne vorangehende Oberflächenbehandlung erfolgen (J. Colloid Interface Sci. 183, 18-25 (1996), Chem. Mater. 8, 1575-1578 (1996), J. Chem. Phys. 99, 13065-13069 (1995)). Auf diese Weise wird jedoch ebenfalls eine geladene Oberfläche mit ihren inhärenten Nachteilen erzeugt. Außerdem ist die Resistenz solcher ausschließlich über hydrophobe Wechselwirkungen adsorbierter Systeme gegenüber verschiedenen Lösungsmitteln sehr begrenzt und ihre mechanische Stabilität nicht gewährleistet (Macromol. Chem. Phys. 198, 3239 (1997)).

Mit den bisher angewendeten Verfahren, wie der Dextranisierung von Oberflächen (Biosensors und Bioelectronics 11, 579 (1996), Journal of Biomedical Materials Research 18, 953 (1984)), konnten unbehandelte Kunststoffoberflächen nicht beschichtet werden. Auch konnte in den Verfahren auf organische Lösungsmittel nicht gänzlich verzichtet werden, was jedoch im Hinblick auf die Umweltverträglichkeit der Verfahren bedenklich ist und zusätzlich Kosten verursacht. Bei der Funktionalisierung mittels Epoxygruppen (Journal of Biomedical Materials Research 18, 953 (1984)) müssen außerdem die verwendeten Polymerlösungen eine sehr hohe Konzentration aufweisen, um zu befriedigenden Ergebnissen zu kommen. Dies führt jedoch einerseits zu schwer handhabbaren Lösungen aufgrund ihrer hohen Viskosität und andererseits zu einer Erhöhung der Kosten. Bei der Oberflächenmodifikation mit Aminodextran und EDC (Biosensors and Bioelectronics 11, 579 (1996)) sind teure Zusatzreagenzien erforderlich und die Beschichtung muß Aminogruppen enthalten, deren Fähigkeit zu elektrostatischer Wechselwirkung eine inhärente Störungsquelle darstellt.

C. Furnier et al. beschreiben in Langmuir **1995**, *11,* 2344-2347 die Beschichtung von Polystyrol-Divinylbenzol-Partikeln mit modifiziertem Dextran. Um die Absorption zu fördern, wurde das Polysaccharid vor der Absorption zunächst mit niedrigen Konzentrationen von Phenoxygruppen hydrophobisiert.

Aufgabe der vorliegenden Erfindung ist es, Gegenstände mit durch eine Beschichtung modifizierten hydrophoben oder hydrophobisierten Oberflächen, wie solchen aus Kunststoffen, Glas, Quarz, Silizium, Silber oder Gold, bereitzustellen, die im wesentlichen keiner unspezifischen Adsorption von hydrophoben oder geladenen Stoffen unterliegen.

Reine Glas-, Quarz-, Silizium-, Silber- oder Goldoberflächen sind zwar in völlig sauberem Zustand an sich nicht hydrophob, neigen jedoch dazu, z.B. in der Raumluft vorhandene organische Verunreinigungen zu adsorbieren und so hydrophob zu werden. Diese unspezifische Adsorption gilt es zu verhindern. Daneben soll durch entsprechende Beschichtung auch eine unspezifische Ladungswechselwirkung bei Glas-, Quarzund Siliziumoberflächen unterbunden werden.

Eine weitere Aufgabe der Erfindung ist es, Beschichtungen der genannten Art bereitzustellen, die sich weiter funktionalisieren lassen und somit zu einer Verwendung in biotechnologischen Geräten geeignet sind.

Rezeptoren von Biosensoren auf Affinitätsbasis (Affinitätssensoren) müssen z.B. so modifiziert werden, daß an ihnen aus einem komplexen Vielkomponentensystem selektiv nur ein bestimmter Stoff adsorbiert wird. Die adsorbierte Stoffmenge kann dann über Oberflächenplasmonenresonanz vermessen werden.

Bei der Affinitätschromatographie muß das aktive Trennmedium so geartet sein, daß nur die zu analysierende Substanz selektiv gebunden wird, während unspezifische Adsorption so weit wie möglich unter Verwendung der erfindungsgemäßen Beschichtung zurückgedrängt wird.

Bei der Vermessung von sehr kleinen zu analysierenden Materialmengen, z.B. im Mikroliterbereich und darunter, ist es von größter Wichtigkeit, daß das zu analysierende Material nicht an Gefäßwänden und Schlauchinnenseiten haften bleibt und damit signifikante Anteile verloren gehen. Es ist also gewünscht, die Adsorption des zu analysierenden Materials zu minimieren. In Gefäßen und Schläuchen, die mit der erfindungsgemäßen Beschichtung ausgekleidet sind, kann so die unspezifische Adsorption unterdrückt werden.

Außerdem kann die erfindungsgemäße Beschichtung nach weiterer Funktionalisierung auch als Matrix für Festphasensynthesen dienen.

Eine weitere Aufgabe ist es, ein Verfahren bereitzustellen, das das Aufbringen dieser Beschichtungen auf hydrophobe oder hydrophobisierte Oberflächen in einfacher und kostengünstiger, jedoch umweltfreundlicher Weise ermöglicht und zu sowohl mechanisch widerstandsfähigen als auch gegenüber verschiedenen Lösungsmitteln resistenten Beschichtungen führt.

Die vorstehenden Aufgaben werden gelöst durch das Beschichten von hydrophoben oder hydrophobisierten Oberflächen mit hydrophilen ungeladenen Polymeren, die hydrophobe Gruppen aufweisen.

Im einzelnen stellt die vorliegende Erfindung einen Gegenstand mit einer hydrophoben oder hydrophobisierten Oberfläche und einer darauf aufgebrachten Beschichtung aus einem Polysaccharid, das hydrophobe Gruppen aufweist, zur Verfügung.

Weiterhin wird ein Verfahren zur Herstellung des Gegenstandes zur Verfügung gestellt, das dadurch gekennzeichnet ist, daß man eine zu beschichtende hydrophobe Oberfläche des Gegenstands in eine Lösung eines Polysaccharids mit hydrophoben Gruppen taucht, bzw. eine zu beschichtende Glas-, Quarz-, Silizium- oder Goldoberfläche zunächst hydrophobisiert und danach in die Lösung des Polysaccharids taucht.

Gegenstände der Erfindung mit der durch die aufgebrachte Beschichtung modifizierten Oberfläche können als biotechnologische Matrix oder Instrument verwendet werden oder als Matrix für die biochemische Festphasensynthese, z.B. Festphasenproteinsynthese, eingesetzt werden.

Zur Beschichtung wird ein Polysaccharid, das hydrophobe Gruppen aufweist, verwendet. Bevorzugt ist ein Polysaccharid aus Glucoseeinheiten. Stärker bevorzugt ist Dextran, Pullulan oder Inulin, besonders bevorzugt ist Dextran. Die hydrophoben Gruppen des Polysaccharids ermöglichen durch ihre hydrophobe Charakteristik im wesentlichen die Adsorption an den zu funktionalisierenden Oberflächen, indem ein überwiegender Teil der hydrophoben Gruppen im Polymer zur hydrophoben oder hydrophobisierten Oberfläche weist und sich so hydrophobe Wechselwirkungen zwischen den hydrophoben Gruppen und der Oberfläche ausbilden. Das Polymer ist also vorwiegend über hydrophobe Wechselwirkungen an die Oberfläche-gebunden. Bevorzugt sind die hydrophoben Gruppen vernetzbar. Stärker bevorzugt sind hydrophobe Gruppen, die durch BeStrahlung z.B. mit UV-Licnt vernetzt werden können. Eine besonders bevorzugte hydrophobe Gruppe ist die 4-Vinylbenzylgruppe, die z.B. über partielle Veretherung von 4-Vinylbenzylchlorid mit Hydroxygruppen des Polymers in dieses eingeführt werden kann. Sie kann durch Bestrahlung mit UV-Licht der. Wellenlänge 254 nm vernetzt werden. Besteht die Beschichtung aus Dextran, können vorzugsweise 1 bis 20 % der verfügbaren Hydroxylgruppen durch 4-Vinylbenzylgruppen substituiert werden.

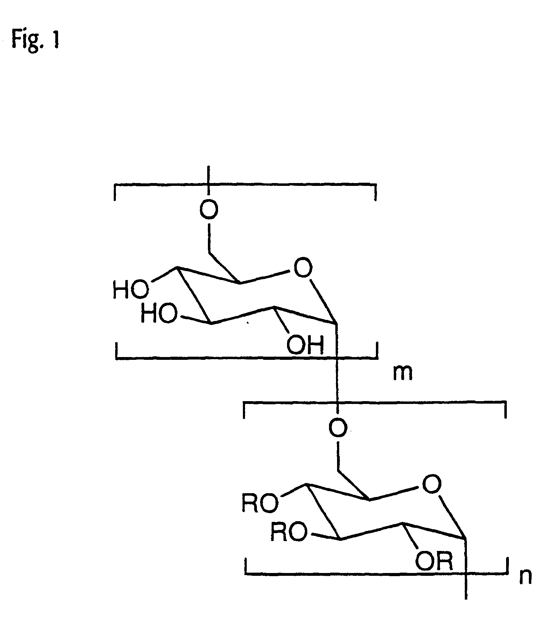

Fig. 1 zeigt eine bevorzugte Ausführungsform eines Polysaccharids. Das Verhältnis vom m zu n reicht von 5:1 bis 20:1. R wird ausgewählt aus Wasserstoff und einer hydrophoben Gruppe, z.B. der 4-Vinylbenzylgruppe mit der Maßgabe, daß wenigstens ein R eine hydrophobe Gruppe ist.

Bei dem Polysaccharid in Fig. 1 handelt es sich um ein statistisches Copolymer, d.h. die Abfolge der Monomereinheiten ist zufällig.

Die erfindungsgemäße Beschichtung kann auf unterschiedlichste Oberflächen aufgebracht werden, wobei zwischen hydrophoben und hydrcphobisierten Oberflächen unterschieden wird.

Bei den hydrophoben Oberflächen kann es sich um Oberflächen von Kunststoffen, z.B. Polyethylen, Polypropylen, Polycarbonat (z.B. Makrolon®) und thermoplastische Olefinpolymere amorpher Struktur (z.B. Topas®) handeln. Bei den hydrophobisierten Oberflächen kann es sich um Silber-, Gold-, Glas-, Quarz- oder Siliziumoberflächen handeln.

Handelt es sich um Glas-, Quarz- oder Siliziumoberflächen, so sind diese hydrophobisiert, d.h. daß die Oberflächen einer hydrophobisierenden Behandlung unterzogen wurde. Diese kann beispielsweise mit einem Polykation, das hydrophobe Gruppen aufweist, erfolgen. Erst nach der Hydrophobisierung folgt dann die Adsorption des Polysaccharids. Alternativ können auch andere Verfahren zur Hydrophobisierung angewendet werden, z.B. eine Behandlung mit Alkoxyalkylsilanen. Diese sind jedoch meist komplizierter und langwieriger als die Adsorption eines Polykations mit hydrophoben Gruppen. Bei Silber- oder Goldoberflächen kann eine Hydrophobisierung durch Behandlung mit einem Alkylthiol oder mit einem negativ geladenen Thiol, wie Mercaptopropylsulfonat, auf das dann wiederum ein Polykation mit hydrophoben Gruppen aufgebracht werden kann, erreicht werden.

Vorzugsweise wird ein Polykation zur Hydrophobisierung eingesetzt, das über die hydrophoben Gruppen vernetzbar ist. Die Vernetzung wird vorzugsweise durch Strahlung bewirkt. Bei den hydrophoben Gruppen des Polykations handelt es sich bevorzugt um 4-Vinylbenzylgruppen. Eine besonders bevorzugte Ausführungsform des Polykations mit hydrophoben Gruppen ist Poly(methacrylsäure-(3-dimethylaminopropylamid)), das zu 100 % mit 4-Vinylbenzylchlorid quaternisiert wurde.

Eine Vernetzung der Beschichtung über die hydrophoben Gruppen des Polysaccharids sowie eine Vernetzung des gegebenenfalls vorhandenen vernetzbaren Polykations über die hydrophoben Gruppen erhöht sowohl die mechanische Stabilität als auch die Lösungsmittelresistenz der Beschichtung. Wird die Vernetzung durch Strahlung bewirkt, so ist es auch möglich, nur Teilbereiche der Beschichtung und des Polykations zu vernetzen. Dazu werden strahlungsundurchlässige Masken verwendet, die den nicht zu vernetzenden Bereich abdecken.

Zusätzlich ist eine Funktionalisierung der Beschichtung sowohl über die Hydroxylgruppen als auch über die hydrophoben Gruppen, die nicht vernetzt werden, möglich.

Ein Teil der Hydroxylgruppen kann beispielsweise mit Bromessigsäure zur Reaktion gebracht werden, wodurch Carboxylgruppen in die Beschichtung eingeführt werden, die wiederum einer Veresterung oder Amidierung unterworfen werden können.

Diese Verfahrensweise kann insbesondere bei der Festphasenproteinsynthese von Nutzen sein.

Des weiteren können über radikalische Reaktionen an den nicht vernetzten 4-Vinylbenzylgruppen Thiogruppen eingeführt werden.

Die funktionalisierten Beschichtungen können insbesondere zur Herstellung von Biosensoren oder für weitere Anwendungen in der Diagnostik oder bei Screening-Verfahren eingesetzt werden.

Zur Herstellung des beschichteten Gegenstandes, dessen beschichtete Oberfläche im wesentlichen keiner unspezifischen Adsorption hydrophober oder geladener Stoffe unterliegt, wird die hydrophobe oder hydrophobisierte Oberfläche des Gegenstandes lediglich in eine Lösung eines Polysaccharides mit hydrophoben Gruppen getaucht. Vorzugsweise handelt es sich um eine wäßrige Lösung. Die Eintauchdauer kann zwischen 8 und 48 h liegen und beträgt typischerweise ca. 24 h. Das Verfahren kann in einem Temperaturbereich von 1 bis 50°C, bevorzugt bei Raumtemperatur, durchgeführt werden. Die Konzentration des Polysaccharids in der Lösung reicht von 0,001 bis 0,1 mol·l⁻¹, bevorzugt von 0,01 bis 0,05 mol·l⁻¹ und ist besonders bevorzugt 0,02 mol·l⁻¹. Enthält das Polysaccharid vernetzbare hydrophobe Gruppen, folgt vorzugsweise eine Bestrahlung, wodurch die Gruppen vernetzen und so die mechanische Stabilität der Beschichtungen erhöht wird. Die Art der zu verwendenden Oberflächen sowie des Polysaccharids wurden bereits oben im Detail beschrieben.

Wie weitere Untersuchungen zeigten, eignet sich dieses einfache Verfahren zum Aufbringen der erfindungsgemäßen Beschichtung nicht nur für Polysaccharide, sondern ganz generell für ungeladene hydrophile Polymere mit hydrophoben Gruppen.

Die vorliegende Erfindung stellt somit ein Verfahren zur Herstellung einer Beschichtung, die im wesentlichen keiner unspezifischen Adsorption hydrophober Stoffe unterliegt, auf einer Oberfläche eines Gegenstandes zur Verfügung, das dadurch gekennzeichnet ist, daß man die Oberfläche, gegebenenfalls nach vorangehender Hydrophobisierung, in eine Lösung eines ungeladenen hydrophilen Polymers mit hydrophoben Gruppen taucht.

Bei dem ungeladenen hydrophilen Polymer handelt es sich vorzugsweise um Polymere mit Hydroxygruppen oder Polyoxyalkylengruppen. Die hydrophoben Gruppen und deren bevorzugte Ausführungsformen sind die vorstehend beschriebenen. Stärker bevorzugte Polymere mit Hydroxygruppen sind Polysaccharide der oben beschriebenen Art. Die Art der Polymere mit Polyoxyalkylengruppen ist nicht besonders beschränkt. Beispielsweise können Polyalkylenderivate wie Poly(meth)acrylsäurederivate, z.B. Poly(meth)acrylamid oder Poly(meth)acrylsäureester, verwendet werden. Die Polyoxyalkylengruppen sind gegebenenfalls über die Carboxy- oder Amidgruppen der Polymere als Seitenketten an das Polymergrundgerüst gebunden.

Als Polyoxyalkylengruppen eignen sich besonders gut Polyoxyethylengruppen.

Besonders bevorzugte Polymere sind die Verbindungen mit den folgenden Formeln I, II, III und IV 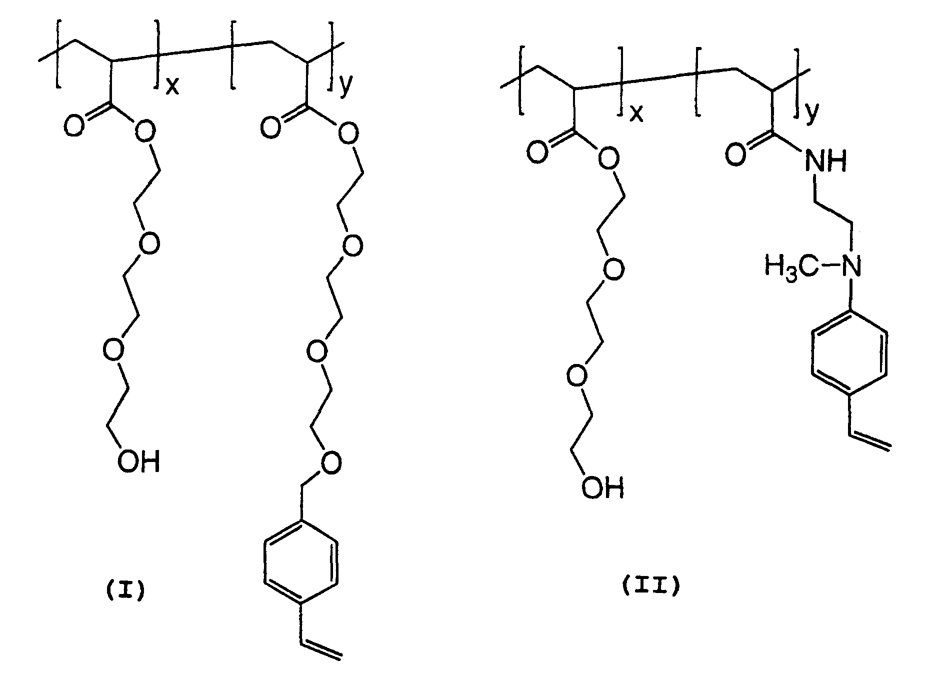 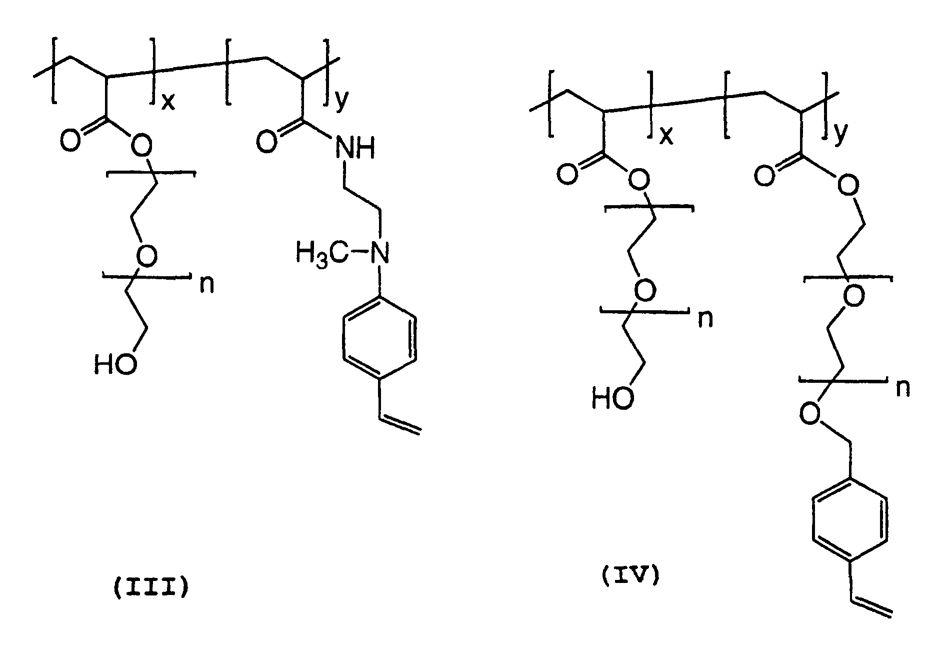 in denen x:y von 20:1 bis 5:1 reicht und n von ca. 50 bis ca. 1000 reicht.

Die Erfindung wird durch die folgenden Beispiele weiter erläutert.

### BEISPIELE

### Bezugsbeispiel

### Synthese von 4-Vinylbenzyl-substituiertem Dextran (VBD):

2,00 g Dextran (Dextran T500 von Pharmacia, 37 mmol Hydroxylgruppen) werden in einer Mischung aus 40 ml Wasser und 10 ml Dimethylsulfoxid gelöst und mit 0,384 g Kaliumhydroxid (6, 74 mmol) versetzt. Wenn diese gelöst sind, werden 1,029 g 4-Vinylbenzylchlorid und 2 Tropfen Nitrobenzol zugegeben. Die Mischung wird für 4 Tage bei Raumtemperatur gerührt. Anschließend wird das Polymer in 400 ml Aceton gefällt, wobei unter Lichtausschluß gearbeitet werden muß.
Ausbeute: 2,04 g (98%)
¹H-NMR-Spektrum (D₂O): 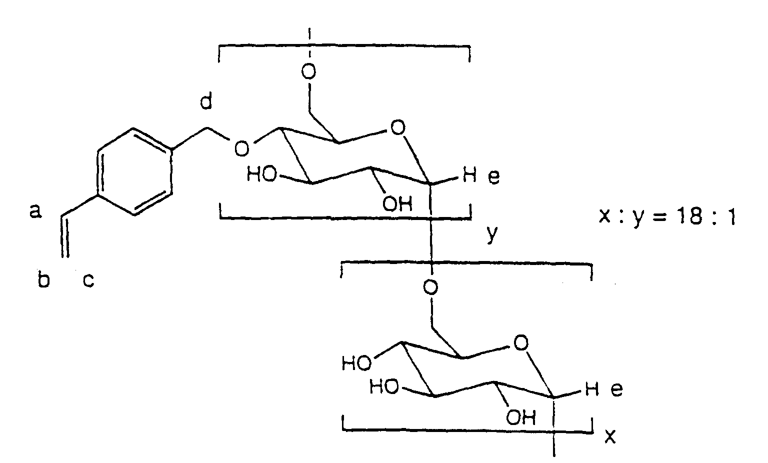 5 [ppm]:
7,55 - 7,25 (m, Hₐᵣₒₘ), 6,85 - 6,65 (m, Hₐ), 5,8 (m, H_{c}), 5,3 (m, H_{b}), 4,92 (Hₑ), 4,55 (H_{d}, teilweise durch HDO verdeckt), 4,2 - 3,3 (mehrere m, H-Atome des Polysaccharidgerüstes)

Die Abfolge der Monomereinheiten ist zufällig, es handelt sich also um ein statistisches Copolymer. Die Funktionalisierung mit der hydrophoben Gruppe erfolgt nicht ausschließlich an den 4-Positionen der Saccharid-Einheiten, sondern mehr oder minder statistisch an allen verfügbaren Hydroxylgruppen.

### Beispiel 1

### Beschichtung von Kunststoffen

Im ersten Schritt wird die zu beschichtende Kunststoffoberfläche durch Eintauchen in konzentriere Schwefelsäure für ca. 30 s gereinigt. Danach wird dreimal mit demineralisiertem Wasser gespült und anschließend wird der Kunststoff für 24h in eine wäßrige Lösung VBD (2·10⁻² mol·l⁻¹) getaucht. Anschließend wird dreimal mit demineralisiertem Wasser gespült. Dann wird die Photovernetzung der aufgebrachten Schicht durch UV-Bestrahlung bei 254 nm für 60 s vorgenommen.

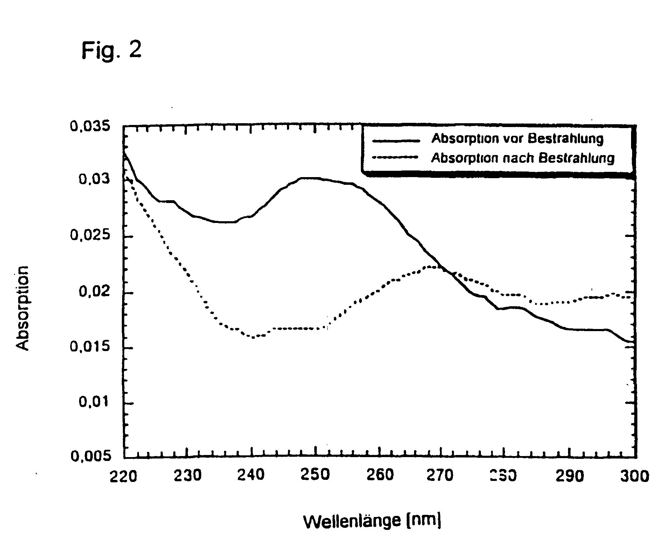

Fig. 2 zeigt UV-Spektren einer Schicht des photoreaktiven Polymers auf Topas®. Der Erfolg der Photoreaktion ist durch die Abnahme der Absorption bei 254 nm dokumentiert (gestrichelte Meßkurve).

Vergleichende Messungen unspezifischer Adsorptionsvorgänge wurden mit einem Konfokalmikroskop durchgeführt. Hierbei wird die Oberfläche in Kontakt mit einer Lösung eines fluoreszenzmarkierten Proteins gebracht. Mit Hilfe des Mikroskops wird die Proteinkonzentration als Funktion des Abstandes von der Oberfläche aufgenommen, indem die Fluoreszenzintensität in der jeweiligen Fokalebene bestimmt wird. Man erhält ein nahezu gleichmäßiges Helligkeitsprofil in Abhängigkeit vom Oberflächenabstand. Dies deutet darauf hin, daß an der funktionalisierten Oberfläche keine unspezifische Adsorption erfolgt.

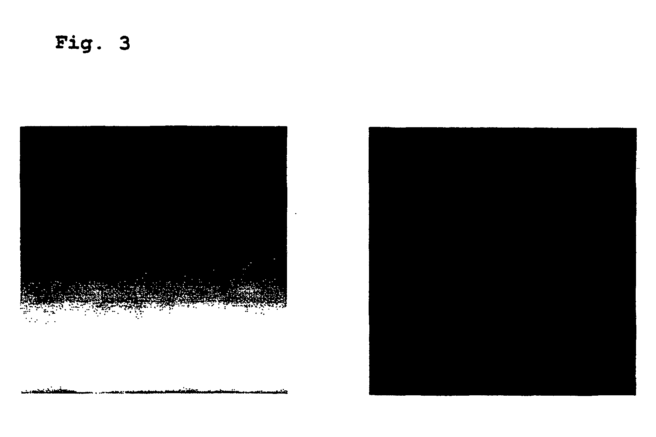

Die in Fig. 3 links abgebildete Oberfläche stellt das Konzentrationsprofil einer 500 nmolaren fluoreszenzmarkierten Rinderserumalbumin-(BSA-Texas Red)-Lösung, auf einer beschichteten Topas®-Oberfläche, aufgenommen mit Konfokalmikroskopie dar, wobei helle Bereiche hohen BSA-Konzentrationen entsprechen. Rechts in Fig. 3 ist die unbeschichtete Referenzoberfläche abgebildet. Der abgebildete vertikale Bereich entspricht etwa 2 µm.

### Beispiel 2

### Beschichtung von Silber-, Gold-, Glas-, Quarz- oder Siliziumoberflächen

Die Oberflächen werden nach allgemein üblichen Methoden gereinigt (Ber. Bunsenges. phys. Chem. 100, 1033 (1996), Journal of Biomedical Materials Research 18, 953 (1984)). Die gereinigten Gold- oder Silberoberflächen werden dann ca. 12 h lang in eine wäßrige Mercaptopropylsulfonatlösung (2·10⁻² mol·l⁻¹ eingetaucht und anschließend für 20 Minuten in eine Lösung (2·10⁻² mol·l⁻¹) von Poly(methacrylsäure-(3-dimethylaminopropylamid)), welches zu 100 % mit 4-Vinylbenzylchlorid quaternisiert wurde, gegeben. Die gereinigten Glas-, Quarz- oder Siliziumoberflächen werden, ohne zuvor mit der Mercaptopropylsulfonatlösung behandelt zu werden, für 20 Minuten in eine Lösung (2·10⁻² mol·l⁻¹) von Poly-(methacrylsäure-(3-dimethylaminopropylamid)), welches zu 100 % mit 4-Vinylbenzylchlorid quaternisiert wurde, gegeben. Nach dreimaligem Spülen mit Reinstwasser wird der Werkstoff für 24 h in eine wäßrige Lösung VBD (2·10⁻² mol·l⁻¹) getaucht. Anschließend wird dreimal mit demineralisiertem Wasser gespült. Dann wird die Photovernetzung der aufgebrachten Schichten durch UV-Bestrahlung bei 254 nm für 60 s vorgenommen.

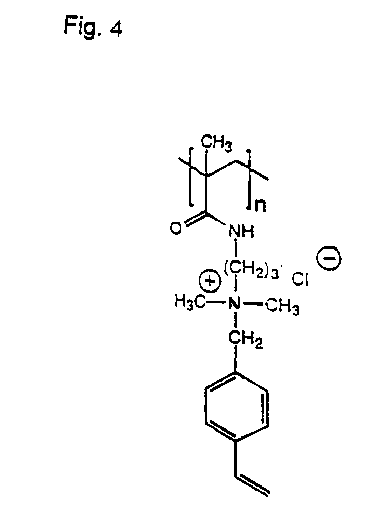

Fig. 4 zeigt die Strukturformel von Poly(methacrylsäure-(3-dimethylaminopropylamid)), quaternisiert mit 4-Vinylbenzylchlorid.

## Patentansprüche

1. Gegenstand mit einer hydrophoben oder hydrophobisierten Oberfläche und einer darauf aufgebrachten Beschichtung aus einem Polysaccharid, das hydrophobe Gruppen aufweist wobei die Beschichtung über die hydrophoben Gruppen durch Strahlung vernetzt ist.

2. Gegenstand nach Anspruch 1, **dadurch gekennzeichnet, daß** das Polysaccharid über hydrophobe Wechselwirkungen an die Oberfläche gebunden ist.

3. Gegenstand nach einem der Ansprüche 1 oder 2, **dadurch gekennzeichnet, daß** das Polysaccharid ein Dextran mit hydrophoben Gruppen ist.

4. Gegenstand nach einem der Ansprüche 1 bis 3, **dadurch gekennzeichnet, daß** die hydrophoben Gruppen 4-Vinylbenzylgruppen sind.

5. Gegenstand nach einem der Ansprüche 1 bis 4, **dadurch gekennzeichnet, daß** die Beschichtung aus einem Dextran besteht, in dem 1 bis 20 % der verfügbaren Hydroxylgruppen durch 4-Vinylbenzylgruppen substituiert sind.

6. Gegenstand nach einem der Ansprüche 1 bis 5, **dadurch gekennzeichnet, daß** die Oberfläche aus einem hydrophoben Kunststoff besteht.

7. Gegenstand nach einem der Ansprüche 1 bis 5, **dadurch gekennzeichnet, daß** die Oberfläche Glas, Quarz, Silizium, Silber oder Gold umfaßt und vor dem Aufbringen des Polysaccharids hydrophobisiert wird.

8. Gegenstand nach Anspruch 7, **dadurch gekennzeichnet, daß** die Hydrophobisierung von Glas-, Quarz- oder Siliziumoberflächen durch Behandlung mit einem Polykation, das hydrophobe Gruppen aufweist, erfolgt.

9. Gegenstand nach Anspruch 7, **dadurch gekennzeichnet, daß** die Hydrophobisierung von Silber- oder Goldoberflächen durch Behandlung mit einem Alkylthiol, oder mit einem negativ geladenen Thiol, auf das dann ein Polykation mit hydrophoben Gruppen aufgebracht wird, erfolgt.

10. Gegenstand nach einem der Ansprüche 8 oder 9, **dadurch gekennzeichnet, daß** das Polykation über die hydrophoben Gruppen vernetzbar ist.

11. Gegenstand nach Anspruch 10, **dadurch gekennzeichnet, daß** die Vernetzung durch Strahlung bewirkt ist.

12. Gegenstand nach einem der Ansprüche 8 bis 11, **dadurch gekennzeichnet, daß** die hydrophoben Gruppen 4-Vinylbenzylgruppen sind.

13. Gegenstand nach einem der Ansprüche 8 bis 12, **dadurch gekennzeichnet, daß** das Polykation mit hydrophoben Gruppen Poly(methacrylsäure- (3-dimethylaminopropylamid)) ist, welches zu 100 % mit 4-Vinylbenzylchlorid quaternisiert wurde.

14. Gegenstand nach einem der Ansprüche 1 bis 13, **dadurch gekennzeichnet, daß** die Beschichtung und gegebenenfalls vorhandenes vernetzbares Polykation in Teilbereichen oder auf der gesamten Oberfläche vernetzt sind.

15. Gegenstand nach einem der Ansprüche 1 bis 14, **dadurch gekennzeichnet, daß** ein Teil der Hydroxylgruppen der Beschichtung funktionalisiert ist.

16. Verfahren zur Herstellung einer Beschichtung, die im wesentlichen keiner unspezifischen Adsorption hydrophober oder geladener Stoffe unterliegt, auf einer Oberfläche eines Gegenstandes, **dadurch gekennzeichnet, daß** man die Oberfläche, gegebenenfalls nach vorangehender Hydrophobisierung, in eine Lösung eines ungeladenen hydrophilen Polymers mit hydrophoben Gruppen taucht und anschließend die Beschichtung über die hydrophoben Gruppen durch Strahlung vernetzt.

17. Verfahren nach Anspruch 16 zur Herstellung eines beschichteten Gegenstandes nach einem der Ansprüche 1 bis 14, **dadurch gekennzeichnet, daß** man eine zu beschichtende hydrophobe Oberfläche des Gegenstandes in eine Lösung eines Polysaccharids mit hydrophoben Gruppen taucht, oder eine zu beschichtende Glas-, Quarz-, Silizium-, Silber- oder Goldoberfläche zunächst hydrophobisiert und danach in die Lösung des Polysaccharids taucht.

18. Verfahren nach einem der Ansprüche 16 oder 17, **dadurch gekennzeichnet, daß** die Lösung eine wäßrige Lösung ist.

19. Verfahren nach einem der Ansprüche 16 bis 18, **dadurch gekennzeichnet, daß** man in der erhaltenen Beschichtung vorhandene vernetzbare Gruppen in Teilbereichen oder auf der gesamten Oberfläche vernetzt.

20. Verwendung eines Gegenstandes nach einem der Ansprüche 1 bis 15 als biotechnologische Matrix oder Instrument.

21. Verwendung eines Gegenstandes nach einem der Ansprüche 1 bis 15 als Matrix für die biochemische Festphasensynthese.

22. Verwendung eines Gegenstandes nach einem der Ansprüche 1 bis 15 als Affinitätssensor.

23. Verwendung eines Gegenstandes nach einem der Ansprüche 1 bis 15 als aktives Trennmedium bei der Affinitätschromatographie.

## Claims

1. An article with a hydrophobic or hydrophobised surface and a coating applied thereto of a polysaccharide which comprises hydrophobic groups, the coating being crosslinkable via the hydrophobic groups by radiation.

2. An article according to claim 1, **characterised in that** the polysaccharide is bound to the surface via hydrophobic interaction.

3. An article according to one of claims 1 or 2, **characterised in that** the polysaccharide is a dextran with hydrophobic groups.

4. An article according to one of claims 1 to 3, **characterised in that** the hydrophobic groups are 4-vinylbenzyl groups.

5. An article according to one of claims 1 to 4, **characterised in that** the coating consists of a dextran, in which 1 to 20 % of the available hydroxyl groups are substituted by 4-vinylbenzyl groups.

6. An article according to one of claims 1 to 5, **characterised in that** the surface consists of a hydrophobic plastics material.

7. An article according to one of claims 1 to 5, **characterised in that** the surface comprises glass, quartz, silicon, silver or gold and is hydrophobised prior to application of the polysaccharide.

8. An article according to claim 7, **characterised in that** the glass, quartz or silicon surfaces are hydrophobised by treatment with a polycation comprising hydrophobic groups.

9. An article according to claim 7, **characterised in that** the silver or gold surfaces are hydrophobised by treatment with an alkylthiol or with a negatively charged thiol to which a polycation with hydrophobic groups is then applied.

10. An article according to one of claims 8 or 9, **characterised in that** the polycation may be crosslinked via the hydrophobic groups.

11. An article according to claim 10, **characterised in that** crosslinking is effected by radiation.

12. An article according to one of claims 8 to 11, **characterised in that** the hydrophobic groups are 4-vinylbenzyl groups.

13. An article according to one of claims 8 to 12, **characterised in that** the polycation with hydrophobic groups is poly(methacrylic acid (3-dimethylaminopropylamide)), which has been 100% quaternised with 4-vinylbenzyl chloride.

14. An article according to one of claims 1 to 13, **characterised in that** the coating and optionally present crosslinkable polycation are crosslinked in places or over the entire surface.

15. An article according to one of claims 1 to 14, **characterised in that** some of the hydroxyl groups of the coating are functionalised.

16. A process for producing a coating, which is not substantially subject to any nonspecific adsorption of hydrophobic or charged substances, on a surface of an article, **characterised in that** the surface is immersed in a solution of an uncharged hydrophilic polymer with hydrophobic groups, optionally after prior hydrophobisation, and then the coating is crosslinked via the hydrophobic groups by radiation.

17. A process according to claim 16 for producing a coated article according to one of claims 1 to 14, **characterised in that** a hydrophobic surface to be coated of the article is immersed in a solution of a polysaccharide with hydrophobic groups, or a glass, quartz, silicon, silver or gold surface to be coated is initially hydrophobised and then immersed in the polysaccharide solution.

18. A process according to one of claims 16 or 17, **characterised in that** the solution is an aqueous solution.

19. A process according to one of claims 16 to 18, **characterised in that** crosslinkable groups present in the coating obtained are crosslinked in places or over the entire surface.

20. Use of an article according to one of claims 1 to 15 as a biotechnological matrix or instrument.

21. Use of an article according to one of claims 1 to 15 as a matrix for biochemical solid-phase synthesis.

22. Use of an article according to one of claims 1 to 15 as an affinity sensor.

23. Use of an article according to one of claims 1 to 15 as an active separation medium in affinity chromatography.

## Revendications

1. Objet ayant une surface hydrophobe ou rendue hydrophobe sur laquelle est appliqué un revêtement constitué d'un polysaccharide comprenant des groupes hydrophobes, le revêtement pouvant être réticulé par l'intermédiaire des groupes hydrophobes par exposition à un rayonnement.

2. Objet selon la revendication 1, **caractérisé en ce que** le polysaccharide est lié à la surface par des interactions hydrophobes.

3. Objet selon l'une des revendications 1 ou 2, **caractérisé en ce que** le polysaccharide est un dextrane contenant des groupes hydrophobes.

4. Objet selon l'une des revendications 1 à 3, **caractérisé en ce que** les groupes hydrophobes sont des groupes 4-vinylbenzyle.

5. Objet selon l'une des revendications 1 à 4, **caractérisé en ce que** le revêtement est constitué d'un dextrane dans lequel 1 à 20 % des groupes hydroxyle disponibles sont substitués par des groupes 4- vinylbenzyle.

6. Objet selon l'une des revendications 1 à 5, **caractérisé en ce que** la surface est constituée d'une matière plastique hydrophobe.

7. Objet selon l'une des revendications 1 à 5, **caractérisé en ce que** la surface comprend du verre, du quartz, du silicium, de l'argent ou de l'or et est rendue hydrophobe avant l'application du polysaccharide.

8. Objet selon la revendication 7, **caractérisé en ce que** la surface de verre, de quartz ou de silicium est rendue hydrophobe par traitement avec un polycation contenant des groupes hydrophobes.

9. Objet selon la revendication 7, **caractérisé en ce que** la surface d'argent ou d'or est rendue hydrophobe par traitement avec un alkylthiol ou avec un thiol chargé négativement, suivi de l'application d'un polycation ayant des groupes hydrophobes.

10. Objet selon l'une des revendications 8 ou 9, **caractérisé en ce que** le polycation est réticulable par l'intermédiaire des groupes hydrophobes.

11. Objet selon la revendication 10, **caractérisé en ce que** la réticulation s'effectue par exposition à un rayonnement.

12. Objet selon l'une des revendications 8 à 11, **caractérisé en ce que** les groupes hydrophobes sont des groupes 4-vinylbenzyle.

13. Objet selon l'une des revendications 8 à 12, **caractérisé en ce que** le polycation contenant des groupes hydrophobes est un poly(3-diméthylaminopropylamide d'acide méthacrylique) quaternisé jusqu'à 100 % avec du chlorure de 4-vinylbenzyle.

14. Objet selon l'une des revendications 1 à 13, **caractérisé en ce que** le revêtement et le polycation réticulable éventuellement présent sont réticulés dans des zones particulières ou sur toute la surface.

15. Objet selon l'une des revendications 1 à 14, **caractérisé en ce qu'**une partie des groupes hydroxyle du revêtement est fonctionnalisée.

16. Procédé de préparation d'un revêtement, ne subissant essentiellement aucune adsorption non spécifique de substances hydrophobes ou chargées, sur une surface d'un objet, **caractérisé en ce que**, éventuellement après l'avoir préalablement rendue hydrophobe, on plonge la surface dans une solution d'un polymère hydrophile non chargé contenant des groupes hydrophobes, puis on réticule le revêtement par l'intermédiaire des groupes hydrophobes en l'exposant à un rayonnement.

17. Procédé selon la revendication 16 pour la préparation d'un objet revêtu selon l'une des revendications 1 à 14, **caractérisé en ce que** l'on plonge une surface hydrophobe à revêtir de l'objet dans une solution d'un polysaccharide ayant des groupes hydrophobes, ou on rend d'abord hydrophobe une surface de verre, de quartz, de silicium, d'argent ou d'or à revêtir, puis on la plonge dans la solution du polysaccharide.

18. Procédé selon l'une des revendications 16 ou 17, **caractérisé en ce que** la solution est une solution aqueuse.

19. Procédé selon l'une des revendications 16 à 18, **caractérisé en ce que** l'on réticule les groupes réticulables présents dans le revêtement obtenu dans des zones particulières ou sur toute la surface.

20. Utilisation d'un objet selon l'une des revendications 1 à 15 comme matrice ou instrument biotechnologique.

21. Utilisation d'un objet selon l'une des revendications 1 à 15 comme matrice pour la synthèse biochimique en phase solide.

22. Utilisation d'un objet selon l'une des revendications 1 à 15 comme capteur par affinité.

23. Utilisation d'un objet selon l'une des revendications 1 à 15 comme milieu de séparation actif en chromatographie d'affinité.
